# EUROPÄISCHE PATENTSCHRIFT

(11) **EP 0 680 959 B1**
(45) Veröffentlichungstag und Bekanntmachung des Hinweises auf die Patenterteilung: **08.07.1998**
(21) Anmeldenummer: 95105910.4
(22) Anmeldetag: 20.04.1995
(51) Int. Cl.: C07D 323/06

(54) **Verfahren zur Trennung von Formaldehyd und Trioxan**
Process for the separation of formaldehyde and trioxane
Procédé pour la séparation de formaldéhyde et de trioxane

(30) Priorität: 02.05.1994 DE 4415332
(43) Veröffentlichungstag der Anmeldung: 08.11.1995
(73) Patentinhaber: Ticona GmbH, 65926 Frankfurt am Main (DE)
(72) Erfinder: Hoffmockel, Michael, Dr., D-65527 Niedernhausen (DE); Mück, Karl-Friedrich, Dr., D-65207 Wiesbaden (DE); Sextro, Günter, Dr., D-65207 Wiesbaden (DE)

(56) Entgegenhaltungen:
- EP-A- 0 583 907
- DE-A- 1 543 815
- DE-A- 1 668 867
- GB-A- 1 150 607

## Beschreibung

Die Erfindung betrifft ein Verfahren zur Trennung von Gemischen aus Formaldehyd und Trioxan, wie sie beispielsweise bei der Trimerisierung von wasserfreiem Formaldehyd in der Gasphase entstehen.

Trioxan kann aus wäßrigen Formaldehydlösungen unter Verwendung von sauren Katalysatoren hergestellt werden. Aus dem Reaktionsgemisch wird das Trioxan durch extraktive und destillative Verfahrensschritte abgetrennt (US-A 2 304 080, GB-B 1 012 372, DE-A 1 668 867, DE-A 1 543 815, EP-A-0 583 907). Die Extraktion läßt sich durch einen Kristallisationsschritt ersetzen (DE-A 3 508 668). Bei diesen Verfahren zur Trennung von Formaldehyd und Trioxan ist stets Wasser anwesend.

Desweiteren sind Verfahren bekannt, welche die Herstellung von Trioxan aus Formaldehyd in der Gasphase ermöglichen (Deutsche Patentanmeldung P 42 44 582.5 vom 31.12.92, deutsche Patentanmeldung P 43 00 138.6 vom 06.01.93, jeweils Titel "Verfahren zur Herstellung von Trioxan"), DE-C 1 593 990, JP-A 59-25387). Die Trimerisierung in der Gasphase hat neben den höheren Umsätzen den Vorteil, daß die energieaufwendige Abtrennung des Wassers aus dem Reaktionsgemisch entfällt. Im Gegensatz zu der Trennung wäßriger Gemische ist eine Trennung nicht-wäßriger Gemische noch nicht beschrieben worden.

Es bestand also die Aufgabe, ein Verfahren zu finden, mit dem das Reaktionsgemisch aus Formaldehyd und Trioxan in Abwesenheit von Wasser getrennt werden kann.

Die Aufgabe wurde durch die Erfindung gelöst. Sie beschreibt ein Verfahren zur Trennung eines gasförmigen Reaktionsgemisches bei der Herstellung von Trioxan, das als Hauptprodukt Formaldehyd und Trioxan enthält, bei dem a) das den Reaktor verlassende wasserfreie gasförmige Gemisch mit einem organischen Lösemittel, dessen Siedepunkt höher als 135°C ist und das gegenüber Formaldehyd und Trioxan inert ist, im Gegenstrom gewaschen wird, in dem sich das Trioxan in überwiegendem Maße löst und das den Formaldehyd in überwiegendem Maße in der Gasphase beläßt, die in den Reaktor zurückgeführt wird, b) das Trioxan gemeinsam mit restlichem Formaldehyd destillativ aus dem Lösemittel über eine Kolonne abgetrieben wird, wobei das Kopfprodukt im Temperaturbereich von 62 bis 100°C, bevorzugt 65 bis 80°C, partiell kondensiert wird, c) das dabei entstehende Kondensat teils als Rücklauf auf die Kolonne gegeben, teils als Produkt abgezogen wird und d) der nicht kondensierte Anteil in den Waschschritt a) zurückgeführt wird.

Der Begriff "in überwiegendem Maße" weist darauf hin, daß unter den Praxisbedingungen naturgemäß keine vollständige Absorption erfolgt, bzw. daß stets auch ein geringer Anteil an Formaldehyd in dem Waschlösemittel gebunden wird. Die optimalen Bedingungen können aber nur von der Praxis abgeleitet werden.
Als Lösemittel sind solche geeignet, deren Siedepunkt ≥ 135°C ist und die inert gegenüber Formaldehyd und Trioxan sind, z.B. gesättigte aliphatische und aromatische Kohlenwasserstoffe und gesättigte aliphatische sowie aromatische Ether und Polyether. Als vorteilhaft haben sich alkylierte Aromaten erwiesen. Besonders gute Ergebnisse wurden mit Ethylbenzol, den verschiedenen Xylolen und Diethylbenzolen erzielt.

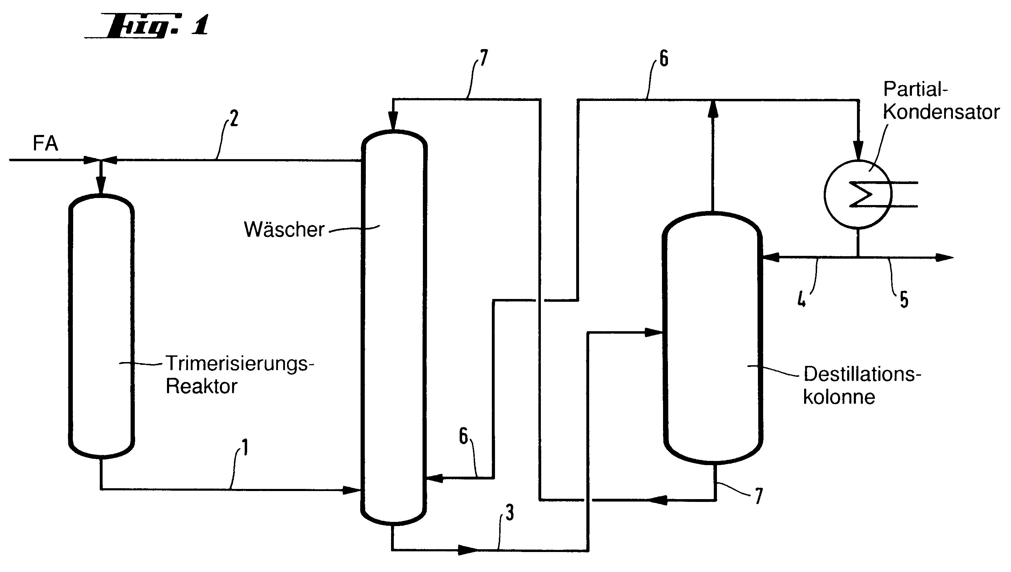

Eine Ausführungsform des Verfahrens (einschließlich des Trimerisierungschrittes) ist in Fig. 1 skizziert. Der wasserfreie Formaldehyd (FA) wird in der Gasphase trimerisiert. Der Produktstrom (1) wird im Gegenstrom mit dem Lösemittel gewaschen. Dabei löst sich im wesentlichen das Trioxan; der Formaldehyd mit Beimengungen von Trioxan und Lösemittel wird zurückgeführt (2) zum Trimerisierungsreaktor. Nach Verlassen des Wäschers wird das Lösemittel mit dem gelösten Trioxan (3) wird in einer Destillationskolonne getrennt. Der Sumpfstrom (7) ist von Trioxan befreit und wird erneut zur Wäsche eingesetzt. Das Kopfprodukt wird partiell kondensiert (Temperaturbereich 62 bis 100°C, bevorzugt 65 bis 80°C). Die nichtkondensierten Anteile werden zurückgeführt (6) zum Wäscher; von den kondensierten Anteilen wird ein Teilstrom als Rücklauf auf die Destillationskolonne gegeben (4) und der andere Teilstrom als Produktstrom (5) abgezogen. Bei Bedarf kann dieser Strom (5) durch weitere Destillationsschritte feingereinigt werden, beispielsweise um den restlichen gelösten Formaldehyd abzutrennen.

### Versuchsbeschreibung:

Es wurden zwei Versuchsreihen durchgeführt.

### Beispiel 1:

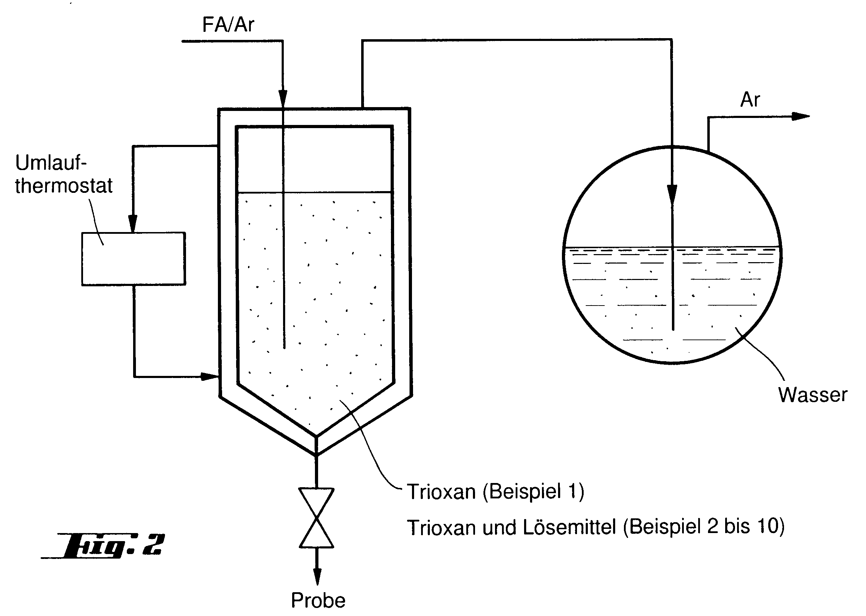

Die Löslichkeit von Formaldehyd in Trioxan und die Zusammensetzung der dazugehörigen Gasphase wurde in einer Apparatur nach Fig. 2 bestimmt. Trioxan wurde in einem thermostatisierten Doppelmantelgefäß mit Bodenablaß vorgelegt. Formaldehyd, hergestellt durch Zersetzung von Cyclohexylhemiformal, wurde durch geschmolzenes Trioxan von 70°C geleitet. Während der Versuchsdauer von 140 Minuten wurden über den Bodenablaß 4 Proben des Trioxans genommen und in der zehnfachen Menge Wassers aufgefangen. Die Proben wurden gaschromatographisch analysiert; dabei wurden 1,3 bis 1,7 Massenprozent Formaldehyd im Trioxan gefunden. Der Gasstrom wurde in Wasser aufgefangen. Im Wasser wurden nach Versuchsende Formaldehyd und Trioxan im Verhältnis 1:1 gefunden. Diese Versuchsanordnung kann als Modell für die Partialkondensation des Kopfstromes der Destillationskolonne dienen (Zusammensetzungen der Ströme (4) und (6)).

### Beispiele 2 bis 12:

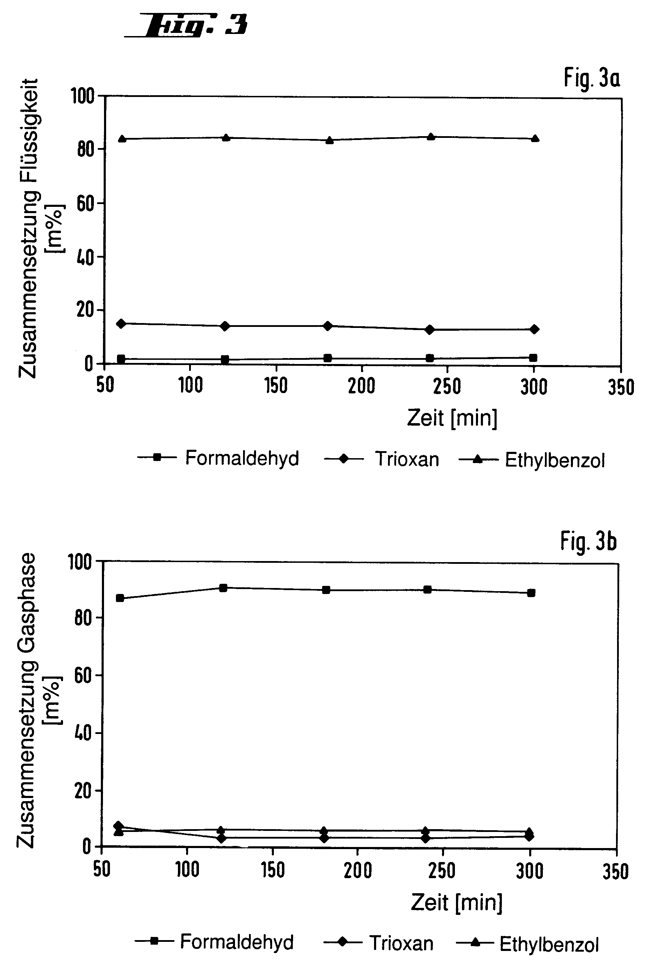

In diesen Versuchen wurden die Gleichgewichtszusammensetzungen, die sich beim Waschen mit dem Lösemittel einstellen, bestimmt. Die Anordnung entspricht derjenigen aus Beispiel 1 (Fig. 2). Bei diesen Versuchen wurde in dem thermostatisierten Gefäß das Lösemittel im Gemisch mit Trioxan vorgelegt. Formaldehyd wurde über eine Versuchsdauer von 5 Stunden eingeleitet. Im Stundenrhythmus wurden Proben von der Flüssigkeit genommen und das Wasser, welches als Absorptionsmittel dient, gewechselt. Sowohl das Wasser als auch die Proben des organischen Lösemittels wurden gaschromatographisch analysiert. Wie in den Fig. 3 a und b gezeigt, ändern sich die Zusammensetzungen der Gasphase und der Flüssigkeit mit der Zeit nur unwesentlich. Die über die Zeit gemittelten Zusammensetzungen, die sich in den Beispielen 2 bis 12 einstellten, sind in Tab. 1 zusammengestellt. Variiert wurden das Lösemittel, die Lösemitteltemperatur sowie die Beladung des Lösemittels mit Trioxan. Diese Versuchsanordnung kann als Modell für die Wäsche mit dem Lösemittel dienen (Zusammensetzungen der Ströme (2) und (3)).

[Anmerkung: In Fig. 2 soll es statt "Trioxan und Lösemittel (Beispiel 2 bis 10)" heißen -- Trioxan und Lösemittel (Beispiel2 bis 12) --. ]

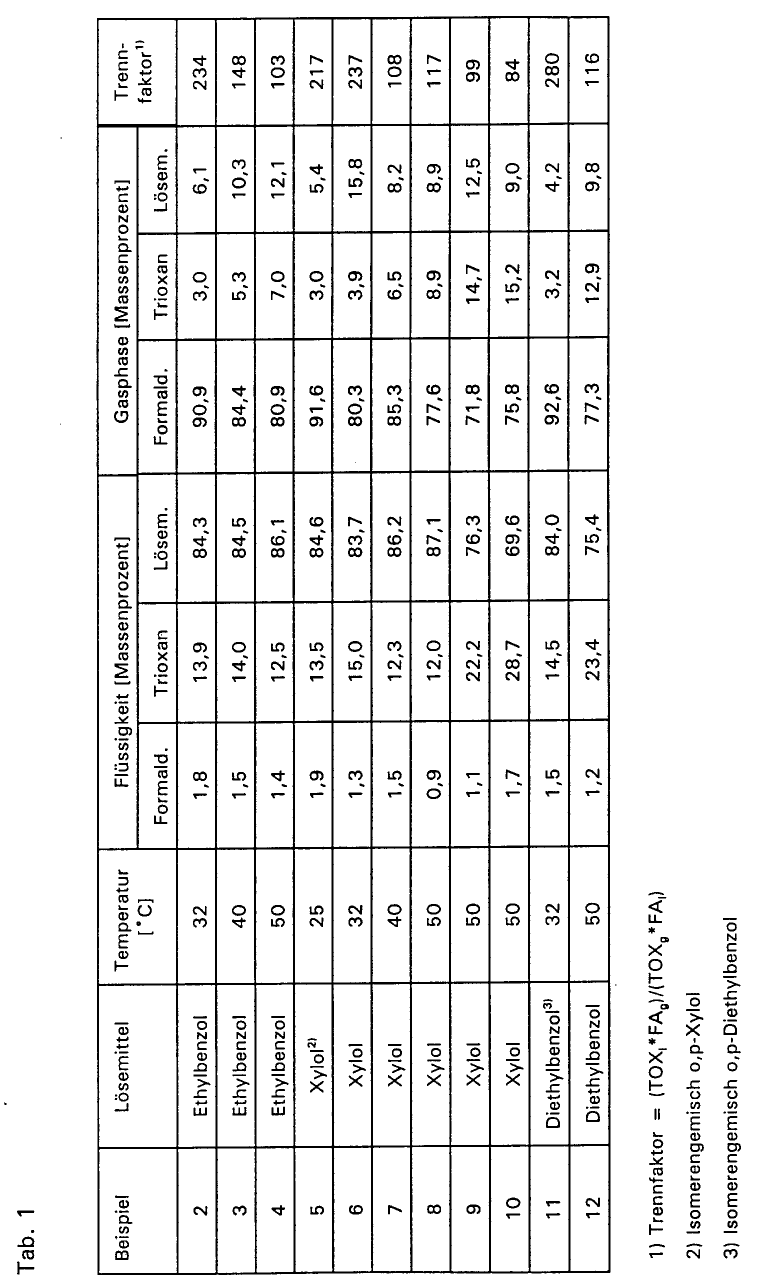

## Patentansprüche

1. Verfahren zur Trennung eines gasförmigen Reaktionsgemisches bei der Herstellung von Trioxan, das als Hauptprodukt Formaldehyd und Trioxan enthält, bei dem a) das den Reaktor verlassende wasserfreie gasförmige Gemisch mit einem organischen Lösemittel, dessen Siedepunkt höher als 135°C ist und das gegenüber Formaldehyd und Trioxan inert ist, im Gegenstrom gewaschen wird, in dem sich das Trioxan in überwiegendem Maße löst und das den Formaldehyd in überwiegendem Maße in der Gasphase beläßt, die in den Reaktor zurückgeführt wird, b) das Trioxan gemeinsam mit restlichem Formaldehyd destillativ aus dem Lösemittel über eine Kolonne abgetrieben wird, wobei das Kopfprodukt im Temperaturbereich von 62 bis 100°C partiell kondensiert wird, c) das dabei entstehende Kondensat teils als Rücklauf auf die Kolonne gegeben, teils als Produkt abgezogen wird und d) der nicht kondensierte Anteil in den Waschschritt a) zurückgeführt wird.

2. Verfahren nach Anspruch 1, dadurch gekennzeichnet, daß der Temperaturbereich 65 bis 80° C beträgt.

3. Verfahren nach Anspruch 1 oder 2, dadurch gekennzeichnet, daß als Lösemittel gesättigte aliphatische und aromatische Kohlenwasserstoffe oder aliphatische sowie aromatische Ether und Polyether eingesetzt werden.

4. Verfahren nach Anspruch 3, dadurch gekennzeichnet, daß Ethylbenzol, die verschiedenen Xylole und Diethylbenzole eingesetzt werden.

## Claims

1. A process for separating a gaseous reaction mixture in the preparation of trioxane, which mixture contains as main product formaldehyde and trioxane, in which process a) the anhydrous gaseous mixture leaving the reactor is scrubbed in countercurrent with an organic solvent whose boiling point is above 135°C and which is inert towards formaldehyde and trioxane in which the trioxane predominantly dissolves and which leaves the formaldehyde predominantly in the gas phase which is returned to the reactor, b) the trioxane together with residual formaldehyde is stripped from the solvent by distillation via a column, the overhead product being partially condensed in the temperature range from 62 to 100°C, c) some of the resulting condensate is applied to the column as reflux and some is taken off as product and d) the non-condensed portion is returned to the scrubbing step a).

2. The process as claimed in claim 1, wherein the temperature range is 65 to 80°C.

3. The process as claimed in claim 1 or 2, wherein the solvent used is saturated aliphatic and aromatic hydrocarbons or aliphatic and aromatic ethers and polyethers.

4. The process as claimed in claim 3, wherein ethyl-benzene, the various xylenes and diethylbenzenes are used.

## Revendications

1. Procédé pour la séparation d'un mélange réactionnel sous forme gazeuse dans le cadre de la préparation de trioxane, contenant comme principaux produits du formaldéhyde et du trioxane, selon lequel:
a) le mélange gazeux anhydre quittant le réacteur est lavé à contre-courant avec un solvant organique dont le point d'ébullition est supérieur à 135°C, qui est inerte vis-à-vis du formaldéhyde et du trioxane, et qui dissout la plus grande partie du trioxane en laissant la plus grande partie du formaldéhyde dans la phase gazeuse, laquelle est réintroduite dans le réacteur,
b) le trioxane et le formaldéhyde résiduel sont séparés du solvant par distillation en colonne, la fraction de tête étant partiellement condensée dans une plage de température de 62 à 100°C,
c) le condensat ainsi obtenu est en partie réinjecté dans la colonne et en partie soutiré comme produit, et
d) la partie non condensée est réacheminée à l'étape de lavage a).

2. Procédé selon la revendication 1, caractérisé en ce que la plage de température est de 65 à 80°C.

3. Procédé selon la revendication 1 ou 2, caractérisé en ce que l'on utilise comme solvants des hydrocarbures aromatiques et aliphatiques saturés, ou des éthers et polyéthers aromatiques et aliphatiques.

4. Procédé selon la revendication 3, caractérisé en ce qu'on utilise l'éthylbenzène, les différents xylènes et le diéthylbenzène.
